# EUROPEAN PATENT APPLICATION

(11) **EP 3 336 547 A1**
(43) Date of publication of application: **20.06.2018**
(21) Application number: 16203695.8
(22) Date of filing: 13.12.2016
(51) Int. Cl.: G01N 33/574

(54) **NOVEL HUMAN BLADDER CANCER BIOMARKERS AND THEIR DIAGNOSTIC USE**

(71) Applicant: Lionex GmbH, 38126 Braunschweig (DE)
(72) Inventor: Singh, Mahavir, 38124 Braunschweig (DE); Elamin, Ayssar, 38126 Braunschweig (DE); Klunkelfuss, Saskia, 38126 Braunschweig (DE); Oehlmann, Wulf, 30177 Hannover (DE); Stehr, Matthias, 38126 Braunschweig (DE)
(74) Representative: Gramm, Lins & Partner Patent- und Rechtsanwälte PartGmbB

(57) **Abstract**

In a first aspect, the present invention relates to methods for diagnosing or identifying bladder cancer in a subject, like for identifying recurrence of bladder cancer in a subject suffering from bladder cancer including monitoring the progression of bladder cancer in a subject afflicted with bladder cancer. Further a method for determining prognosis for survival of a subject afflicted with bladder cancer or for diagnosing or identifying high risk bladder cancer subjects is described together with a method for monitoring the development, the progress or recurrence of bladder cancer in a subject afflicted with or suffering previously from bladder cancer. Finally a method for predicting a clinical outcome or for determining the treatment course in a subject afflicted with bladder cancer is described, said method includes the determination of the level or amount of at least one of S100A12, KLRF1, PTGDR, MT-TC, and RNU6-135P, JUP, NAMPT, AP001434.2, MYBL1, EEF1DP3, RP11-841020.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5. In addition, the use of a kit in a method according to the present invention is provided.

## Description

In a first aspect, the present invention relates to methods for diagnosing or identifying bladder cancer in a subject, like for identifying recurrence of bladder cancer in a subject suffering from bladder cancer including monitoring the progression of bladder cancer in a subject afflicted with bladder cancer. Further a method for determining prognosis for survival of a subject afflicted with bladder cancer or for diagnosing or identifying high risk bladder cancer subjects is described together with a method for monitoring the development, the progress or recurrence of bladder cancer in a subject afflicted with or suffering previously from bladder cancer. Finally a method for predicting a clinical outcome or for determining the treatment course in a subject afflicted with bladder cancer is described, said method includes the determination of the level or amount of at least one of S100A12, KLRF1, PTGDR, MT-TC, and RNU6-135P, JUP, NAMPT, AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5. In addition, the use of a kit in a method according to the present invention is provided.

### Prior art

Bladder cancer (or more precisely urothelial carcinoma of the urinary bladder, UCB) is the fourth and ninth most common cancer amongst men and women, respectively, in in Europe and North America, with an estimated global prevalence of 2.7 million. In 2012, the new cases estimated around 429.000 where again the men incidence is about 3 times higher than in women. UCB results in significant mortality, with overall 5-years survival rates of only 57%-47% when linked with muscle-invasive disease. In addition to its impact on patients, the disease presents a significant economic burden on health systems with a mean estimated treatment and surveillance cost of about USD 200,000 per patient from the time of diagnosis, making it the most expensive of all human cancers to treat from diagnosis to death.

Currently, most patients experience a similar diagnostics pathway following the occurrence of haematuria. A number of biomarkers have also been utilized for diagnosis and surveillance, but no marker has yet been shown to decrease the need for cystoscopy (Horstmann M (2012), Nephrourol Mon 4: 345-349; Miremami J, Kyprianou N (2014), Int J Mol Sci 15: 23897-23908). This is especially problematic given the high recurrence rate, because of which lifelong surveillance is needed to detect any recurrence as early as possible (Horstmann M (2012), see above). The development of reliable, non-invasive tests could therefore improve not only the diagnosis itself but also the quality of life for patients with bladder cancer history. Especially the detection of biomarkers in bodily fluids has shown a high potential. Few urine based tests have been approved by the US Food and Drug Administration (FDA); while blood based tests do not exist. There is therefore still an urgent need for more novel biomarkers for the detection of UCB in general, and particularly in high-risk populations.

The S100 Protein family consists of 25 members, and the expression of the family has been only described in vertebrates. These proteins are characterized by a low molecular weight (9-13 kDa) and two Ca²⁺ binding sites in the form of EF-hands, one of which is unconventional (N-terminal) and has a 100 times higher Ca²⁺ affinity than the C-terminal canonical binding site. Members of this family differ in length and sequence of the hinge region between the binding sites as well as the C-terminal extension following the C-terminal EF-hand. The functions of S100 Proteins range from the regulation of protein phosphorylation, enzyme activities and transcription factors over the dynamics of cytoskeleton constituents, Ca²⁺ homeostasis and cell growth and differentiation to an involvement in the inflammatory response. It is also shown to be involved in the proinflammatory activity by binding the multi-ligand receptor for advanced glycation end-products (RAGE) on endothelial cells as well as recruitment of monocytes. Recent findings described that S100A12 has binding affinity to CD36, a class B scavenger receptor, and this binding mediated translocation of CD36 to the membrane and finally regulate the lipid transport by direct interaction. Altered S100 protein levels have been linked to a range of diseases, including inflammatory and immune disorders, neurodegenerative disorders and cancer. S100 genes also were shown to play roles in bladder tumorigenesis and progression (Yao R, et al.; Anticancer Res 27: 3051-3058). Using only quantitative PCR (qPCR) expression of mRNA S100A12 was described to be increase in in cancerous tissue of transitional cell carcinoma (TCC) patients (Khorramdelazad H, et al. (2015), Asian Pac J Cancer Prev. 16: 2725-2729). Other study reports urinary canine S100A8/A9 and S100A12 concentrations as candidate marker for canine (TCC) (Heilmann RM, et al., (2014), J Vet Diagn Invest 26: 104-112).

### Summary of the present invention

Although various biomarkers are described in the art, there is still ongoing need to provide biomarkers for UCB diagnosis and progression, in particular, with respect to blood gene expression.

In a first aspect, a method for diagnosing or identifying bladder cancer in a subject comprising
a) determining the level or amount of at least one of S100A12, S100A8, S100A9, KLRF1, PTGDR, MT-TC, RNU6-135P, JUP, and NAMPT in a sample of said subject, and
b) diagnosing or identifying bladder cancer based on the level or amount of at least one of S100A12, S100A8, S100A9, KLRF1, PTGDR, MT-TC, RNU6-135P, JUP, and NAMPT is described.

Further, a method for diagnosing or identifying bladder cancer in a subject not being diagnosed for bladder cancer before comprising
a) determining the level or amount of at least one of S100A12, S100A8, S100A9, KLRF1, PTGDR, MT-TC, and RNU6-135P in a sample of said patient; and
b) diagnosing or identifying for a first time bladder cancer in said subject based on the level or amount of at least one of S100A12, S100A8, S100A9, KLRF1, PTGDR, MT-TC, and RNU6-135P is described.

In addition, a method for identifying recurrence of bladder cancer in a subject previously diagnosed and optionally treated for bladder cancer comprising:
a) determining the level or amount of at least one of S100A12, KLRF1, PTGDR, MT-TC, and RNU6-135P AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 in a sample of said subject; and b) identifying bladder cancer recurrence in said subject based on the level or amount of at least one of S100A12, KLRF1, PTGDR, MT-TC, and RNU6-135P is described.

In another aspect, a method for monitoring the progression of bladder cancer in a subject afflicted with bladder cancer, comprising:
a) detecting the level or amount of S100A12 and/or JUP in a first sample from a subject in a first point in time;
b) determining the level or amount of S100A12 and/or JUP in a second sample from the subject in a second point in time; and
c) comparing the level or amount of S100A12 and/or JUP determined in step a) to the level or amount detected in step b) or to a reference thereto for determining progression of bladder cancer based on the change of said level or amount is described.

Moreover, a method for determining prognosis for survival of a subject afflicted with bladder cancer or for diagnosing or identifying high risk bladder cancer subjects, comprising the step of
a) determining the level or amount of at least one of S100A12 and JUP in a sample of said subject, and
b) classifying said subject into a group of subjects as having a decreased likelihood of survival or being a high risk bladder cancer subject based on the level or amount of at least one of S100A12 and JUP is described.

Furthermore, a method for monitoring the development, progress or recurrence of bladder cancer in a subject afflicted with or suffering previously from bladder cancer, comprising
a) determining the level or amount of S100A12 in a sample of said subject in a first time point and,
b) optionally, determining the level or amount of S100A12 in a second time point and
c) determining the development, progress or relapse of bladder cancer in said subject based on the level or amount determined in step a), optionally, compared to the level or amount detected in step b), or compared to a reference value is described.

Finally, a method for predicting a clinical outcome or for determining the treatment course in a subject afflicted with bladder cancer, comprising
a) determining the level or amount of at least one of S100A12, S100A8, S100A9, KLRF1, PTGDR, MT-TC, RNU6-135P, JUP, and NAMPT in at least one sample of the subject and
b) predicting the clinical outcome or determining the treatment course based on the level or amount of at least one of S100A12, S100A8, S100A9, KLRF1, PTGDR, MT-TC, RNU6-135P, JUP, and NAMPT present in said sample is described.

In another aspect, the use of a kit in a method according to the present invention is provided, said kit comprises means for determining the level or amount of at least one of S100A12, S100A8, S100A9, KLRF1, PTGDR, MT-TC, RNU6-135P, JUP, and NAMPT in a sample, like a body fluid sample or body tissue sample, of the subject to be tested and instructions on how to use said test kit in a method according to any one of the preceding claims.

### Brief description of the drawings

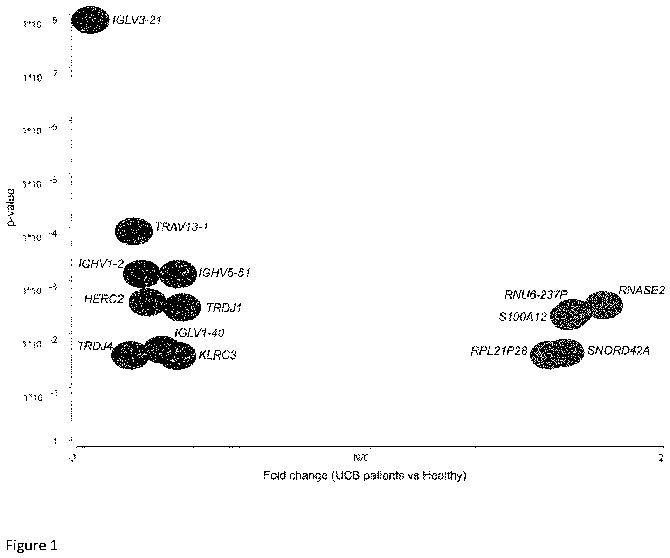

**Fig 1****. Volcano plot of differentially expressed genes in UCB patients against probability.** The figure shows data points of only differentially expressed genes lying above the fdr-corrected p-value <0.05 and a fold change >1.5. Points to the right represent candidates that were upregulated in UCB, while points to the left were downregulated.

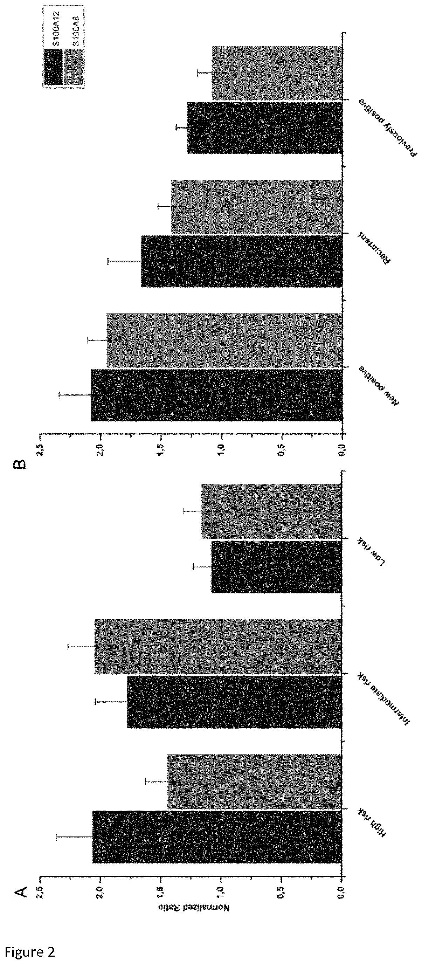

**Fig 2****. qPCR validation of differential expression of S100A12 and S100A8.** Normalized ratio ± ratio error determined for cDNA pools of study risk (A) and prognostic groups (B) by calculation of target/reference ratio of the samples divided by the target/reference ratio of run calibrator (healthy samples). Quantitative PCR was performed for S100A12 (black) and S100A8 (grey).

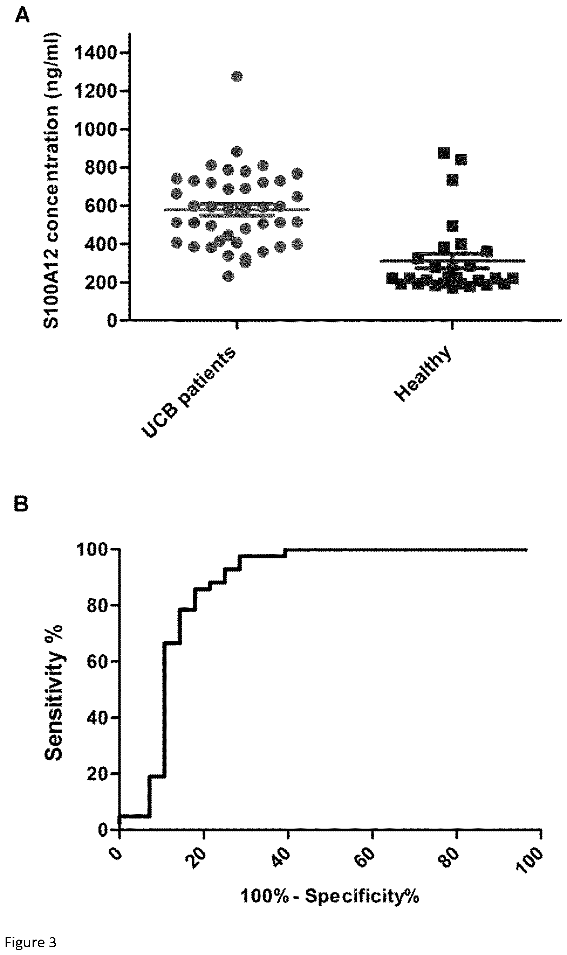

**Fig 3****. Quantitation of S100A12 in UCB plasma samples by BLI.** (A) The concentration of S100A12 was determined by biolayer interferometry (BLI) for plasma samples from each group (UCB patients and Healthy). The mean value for [S100A12] determined by assay is shown for each group and error bars represent the standard error of the mean. (B) An ROC analysis of plasma S100A12 concentrations in all UCB patients vs all healthy in study cohort. Area under the ROC curve is 0.869 with standard error 0.05426 and p-value <0.0001. The 95% confidence interval appeared to be 0.7627 to 0.9754.

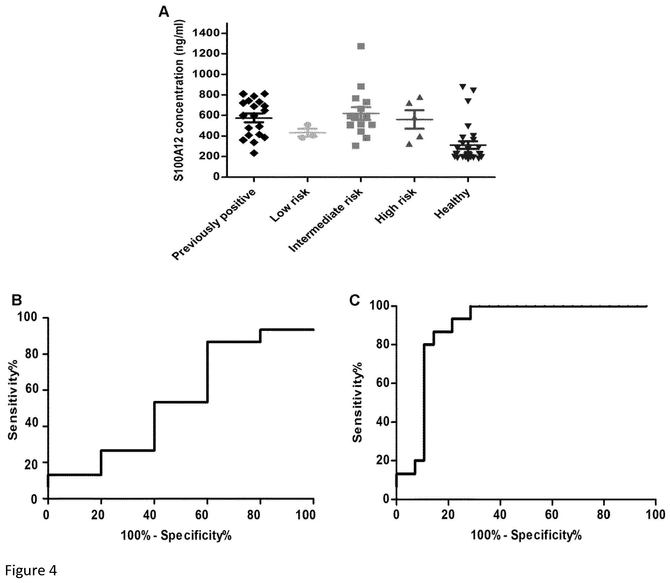

**Fig 4****. A comparison of S100A12 concentrations in plasma within risk groups.** (A) A comparison of S100A12 concentrations in plasma from UCB patients subdivided into groups according to their risk score. The error bars represent the standard error of the mean. (B) An ROC analysis of plasma S100A12 concentrations in high risk UCB patients vs all healthy. Area under the ROC curve is 0.546 with standard error 0.16, p-value <0.76 and the 95% confidence interval appeared 0.2315 to 0.8619. Using an unpaired t-test the difference in the mean value for high risk vs healthy was found statistically significant (p-value=0.047). The best cut-off in this comparison was found to be 422.2ng/ml, which gives a sensitivity of 86.67% and specificity of 40%. (C) An ROC analysis of plasma S100A12 concentrations of intermediate risk UCB patients against study healthy. The area under the ROC curve is 0.888 with standard error 0.052, p-value <0.001 and the 95% confidence interval is 0.785 to 0.991. The t-test showed that difference in the mean value is statistically significant (p-value=0.0002). The best cut-off in this contrast was found to be 372.7ng/ml, which gives a sensitivity of 93.3% and specificity of 78.5%.

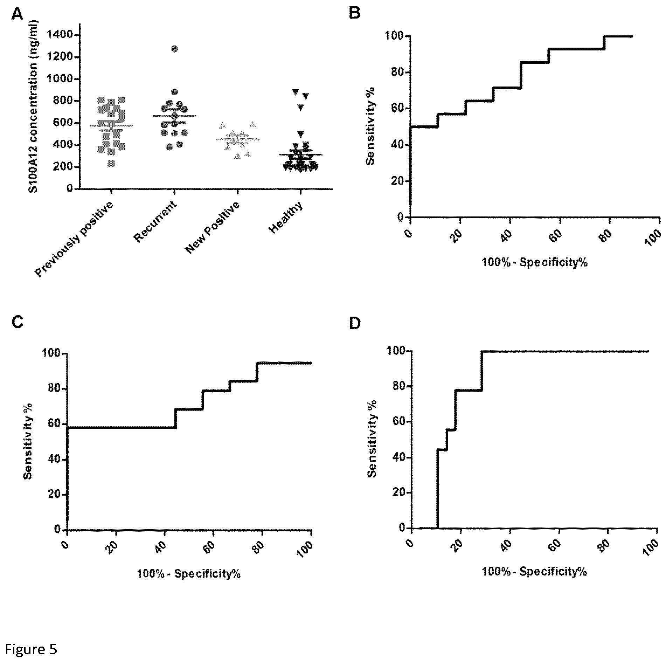

**Fig 5****. Comparison of S100A12 levels in UCB patient prognostics groups.** (A) S100A12 concentrations in plasma from UCB patients sorted according to their prognosis. The error bars represent the standard error of the mean. (B) The ROC analysis of plasma S100A12 concentrations of recurrent group against new positive group of patients. Area under the ROC curve is 0.793 with standard error 0.09, p-value = 0.019 and the 95% confidence interval appeared to be 0.6114 to 0.9759. The best cut-off in this comparison was found to be 512.5ng/ml, which gives a sensitivity of 71.4% and specificity of 66.67%. (C) An ROC analysis of S100A12 concentration of previously positive group (recurrent-negative at sample) compared to new positive group. The area under the ROC curve is 0.725 with standard error 0.095, p-value = 0.058 and 95% confidence interval between 0.5387 and 0.911. Using the cut-off 462.5ng/ml the sensitivity is 68.4% and specificity will be 55.56%. (D) ROC analysis of S100A12 of new positive UCB patients against all healthy. The area under the curve is 0.833 with standard error 0.065, p-value = 0.0002 and the 95% confidence interval is 0.704 to 0.9623. Our showed that best cut-off can be set to be 296.9ng/ml, which gives a sensitivity of 100% and specificity of 71.43%.

### Detailed description of the present invention

The present invention provides new biomarkers in use for methods regarding bladder cancer diagnosis.

That is, in a first aspect, a method for diagnosing or identifying bladder cancer in a subject comprising
a) determining the level or amount of at least one of S100A12, S100A8, S100A9, KLRF1, PTGDR, MT-TC, RNU6-135P, JUP, and NAMPT in a sample of said subject, and
b) diagnosing or identifying bladder cancer based on the level or amount of at least one of S100A12, S100A8, S100A9, KLRF1, PTGDR, MT-TC, RNU6-135P, JUP, and NAMPT is provided.

In a second aspect, a method for diagnosing or identifying bladder cancer in a subject not being diagnosed for bladder cancer before comprising
a) determining the level or amount of at least one of S100A12, S100A8, S100A9, KLRF1, PTGDR, MT-TC, and RNU6-135P in a sample of said patient; and
b) diagnosing or identifying for a first time bladder cancer in said subject based on the level or amount of at least one of S100A12, S100A8, S100A9, KLRF1, PTGDR, MT-TC, and RNU6-135P is provided.

In a further aspect, a method for identifying recurrence of bladder cancer in a subject previously diagnosed and optionally treated for bladder cancer comprising:
a) determining the level or amount of at least one of S100A12, KLRF1, PTGDR, MT-TC, and RNU6-135P AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 in a sample of said subject; and
b) identifying bladder cancer recurrence in said subject based on the level or amount of at least one of S100A12, KLRF1, PTGDR, MT-TC, and RNU6-135P is provided.

Moreover, a method for monitoring the progression of bladder cancer in a subject afflicted with bladder cancer, comprising:
a) detecting the level or amount of S100A12 and/or JUP in a first sample from a subject in a first point in time;
b) determining the level or amount of S100A12 and/or JUP in a second sample from the subject in a second point in time; and
c) comparing the level or amount of S100A12 and/or JUP determined in step a) to the level or amount detected in step b) or to a reference thereto for determining progression of bladder cancer based on the change of said level or amount is provided.

Moreover, in a further aspect, the present invention relates to a method for determining prognosis for survival of a subject afflicted with bladder cancer or for diagnosing or identifying high risk bladder cancer subjects, comprising the step of
a) determining the level or amount of at least one of S100A12 and JUP in a sample of said subject, and
b) classifying said subject into a group of subjects as having a decreased likelihood of survival or being a high risk bladder cancer subject based on the level or amount of at least one of S100A12 and JUP.

In addition, the present invention relates to a method for monitoring the development, progress or recurrence of bladder cancer in a subject afflicted with or suffering previously from bladder cancer, comprising
a) determining the level or amount of S100A12 in a sample of said subject in a first time point and,
b) optionally, determining the level or amount of S100A12 in a second time point and
c) determining the development, progress or relapse of bladder cancer in said subject based on the level or amount determined in step a), optionally, compared to the level or amount detected in step b), or compared to a reference value.

In a further aspect, the present invention relates to a method for predicting a clinical outcome or for determining the treatment course in a subject afflicted with bladder cancer, comprising
a) determining the level or amount of at least one of S100A12, S100A8, S100A9, KLRF1, PTGDR, MT-TC, RNU6-135P, JUP, and NAMPT in at least one sample of the subject and
b) predicting the clinical outcome or determining the treatment course based on the level or amount of at least one of S100A12, S100A8, S100A9, KLRF1, PTGDR, MT-TC, RNU6-135P, JUP, and NAMPT present in said sample.

It has been recognized by the present inventors that various biomarkers including the biomarker S100A12 are suitable for determining and diagnosing different aspects of bladder cancer.

The methods according to the present invention are based on determining the level or amount of at least one of the biomarkers identified herein in a sample of a subject and diagnosing or identifying the desired information based on the level or amount of the at least one biomarker.

As shown below, increase or decrease of said biomarkers allows to arrive at the desired information, namely, the diagnosis of bladder cancer or the recurrence of bladder cancer as well as the monitoring of the progression of bladder cancer and the determination of a prognosis for survival of a subject afflicted with bladder cancer and diagnosing or identifying high risk bladder cancer subjects as well as monitoring the development progress or recurrence of bladder cancer and the prediction of the clinical outcome or monitoring the treatment course in a subject afflicted with bladder cancer accordingly.

As used herein, the term "subject" is preferably a mammal. The mammal can be a human, non-human primate, mouse, rat, dog, cat, horse or cow but are not limited to these examples. A subject can be male or female. In an embodiment, the subject is a human. A subject can be one who has been previously diagnosed with or identified as suffering from bladder cancer and, optionally, but need not have already undergone treatment for bladder cancer. A subject can also be one who has been diagnosed or identified as suffering from bladder cancer but who shows improvement in the disease as a result of receiving one or more treatments for said cancer. Moreover, a subject may also be one who has not been previously diagnosed or identified as having or suffering from bladder cancer. For example, a subject can be one who exhibits one or more risk factors for bladder cancer or a subject who does not exhibit risk factors for bladder cancer or a subject who is asymptomatic for bladder cancer.

The term "determining" as used herein refers to assessing the presence, absence, quantity, level or amount of either a given substance within a clinical or subject derived sample, including qualitative or quantitative concentration levels of said biomarkers or otherwise evaluating the values or amount of a subject's clinical parameter.

The term "comprise" or "comprising of" as well as "contain" or "containing" includes the embodiments of "consist" and "consisting of".

In the context of the present invention, the term "body fluid sample" or "sample of the body fluid" is a biological sample isolated from the subject which can include without being limited thereto, whole blood, serum or plasma as well as urine and nipple aspirate. Preferably, the body fluid is serum or plasma. However, samples useful in the method according to the present invention for diagnostic, prognostic or personalized medicinal uses can be obtained also from surgical samples, such as biopsies or fine needle aspirates, from paraffin embedded tissues, from frozen tumor tissue samples or from fresh tumor tissue samples, from a fresh or frozen body fluid being present in a liquid form.

The term "bladder cancer" is defined to include transitional cell carcinoma or squamous cell carcinoma. The cancer may comprise superficial bladder cancer, invasive bladder cancer, or metastatic bladder cancer. Superficial cancer is only in cells in the lining of bladder and has a high grade of recurrence. A superficial tumor may grow through the lining into the muscular wall into the bladder and become invasive cancer. Invasive cancer can extend through the bladder wall and can grow into a nearby organ such as uterus or vagina (in women) or the prostate gland (in men). It also may invade the wall of the abdomen. The cancer becomes metastatic when it spreads out the bladder into nearby lymph nodes and the organs, such as lung, liver or bones. Various stages of bladder cancer to which the invention is applicable include the high risk of G3 pTa. G3 pT1 an G3 pT2; intermediate risk G2 pTa, G2 pT1; and low risk G1 pTa and G1 pT1, see also table 5 below.

In an embodiment of the present invention, the sample from the subject or for body fluid or body tissue of said subject, in particular, of urine or blood, like whole blood or serum or plasma.

In a further aspect of the present invention, the marker to be detected is the amount or level of S100A12.

The determination may be effected on protein or nucleic acid level. In an embodiment, determination of the level or amount of said biomarker is determined by determining expression on protein level.

Unless otherwise recognized, the term "biomarker" refers to any one of the marker molecules mentioned in the claims, namely, the biomarker being at least one of S100A12, KLRF1, PTGDR, MT-TC, and RNU6-135P AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5.

In an embodiment of the method according to the present invention wherein a clinical outcome or the treatment course is determined or in case of monitoring the development, progress or recurrence of bladder cancer, the S100A12 biomarker is determined.

In an embodiment, the method is an immunological method, in particular, an ELISA or a lateral flow rapid test, also known as a point of care testing. In this connection, the term "point of care testing" also known as bedside-testing is defined as a medical diagnostic testing at or near the point of care, that is, at the time and place of patient care. Point of care tests are simple medical tests and can be performed at the bedside without need of labor work and excessive use of machines. Typically, POCT is often accomplished through the use of transportable, portable, and handle instruments as well as test kits.

In another embodiment, the method is a method wherein the marker is determined on nucleic acid level. The skilled person is well aware of suitable means and methods for effecting the same. These methods include well-known techniques including PCR-techniques etc. For example, the determination is effected quantitatively using quantitative PCR-techniques. For example, the determination is effected by performing a quantitative PCR (qPCR) as described in the art. The skilled person is well aware of other suitable methods for effecting the same.

In case of a detection on protein level, the skilled person is well aware of suitable immunological techniques including techniques with antibodies directed against said biomarker. In one embodiment, protocols for determining the level of protein, including antibodies, antibody fragments, phage display proteins, aptamers, affibodies, chemical ligands, peptides and combinations thereof. The term "antibody" as used herein is intended to include monoclonal antibodies, polyclonal antibodies, and chimeric antibodies. The term "antibody fragment" as used herein is intended to include Fab, Fab', F(ab')₂, scFV, dsFV, ds-scFV, dimers, minibodies, diabodies and multimers thereof and bispecific antibody fragments.

In one embodiment of the invention, the agents or means for determining the biomarker are labeled with a detectable marker or label. The skilled person is well aware of suitable label including fluorophores or chromophores as well as radioisotopes or other suitable label.

In case of the different biomarkers according to the present invention, it has been recognized that an increase or decrease of the same compared to unaffected patients or healthy subjects represents a suitable marker for the methods according to the present invention.

For example, the level or amount of said biomarker is compared to a reference value. In the content of the present invention, the term "reference value" refers to an index value, or a value derived from one or more bladder cancer risk prediction algorisms or computed indices, a value derived from a subject with the same disease or disorder or a value derived from the subject being a healthy subject.

In particular, the reference value is a value from a cohort of healthy subjects.

For example, in case of the biomarker S100A12, an upregulation can be determined in case of patients suffering from bladder cancer compared to healthy subjects as well as between intermediate risk patients and healthy volunteers. Furthermore, in particular with respect to high risk patients a significant upregulation can be observed with highest fold changes compared to healthy subjects. The intermediate risk patients have a lower fold change which dropped further with respect to the low risk patients. The same holds true for prediction of UCB in patients, upregulation is predictive for the recurrence of bladder cancer as well as for monitoring the progression of the bladder cancer and a prognosis for survival of a subject afflicted with bladder cancer or for diagnosing or identifying high risk bladder cancer subjects accordingly. In particular with respect to the method for monitoring the development, progress or recurrence of bladder cancer in a subject afflicted with or suffering previously from bladder cancer, the biomarker S100A12 is particularly useful.

With respect to the other biomarkers, reference is also made to the tables below identifying the up or downregulation of the same in the different methods as claimed herein accordingly.

For example, for RNASE2 an U6-237P-end S100A12 a significant upregulation was observed with respect to bladder cancer compared to healthy volunteers while downregulated genes are IGLV3-21, IGAV1-2 and TRAV13-1. With respect to the other differentially regulated biomarkers, reference is made to table 3 identifying the fold change which means the upregulation (positive fold change) or downregulation (negative fold change) accordingly.

The same holds true for the fold change shown in table 4 of the prognostic biomarkers.

For example for high risk patients the gene JUP was found to be significantly upregulated. Further, upregulation of RNU6-707P and S100A12 can be observed in intermediate risk groups compared to negative controls while TRAJ29 and TRAJ17 are significantly downregulated accordingly.

In a further aspect, the present invention relates to the use of a kit in a method according to any one of the preceding claims, said kit comprises means for determining the level or amount of at least one of S100A12, S100A8, S100A9, KLRF1, PTGDR, MT-TC, RNU6-135P, JUP, AP001434.2, MYBL1, EEF1DP3, RP11-841020.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT in a sample, like a body fluid sample or body tissue sample, of the subject to be tested and instructions on how to use said test kit in a method according to any one of the preceding claims.

The kits for determining the level or amount of said biomarkers are well-known to the skilled person including means for determining the same on protein level as well as means for determining the same on nucleic acid level. For example, the means on protein level include antibodies etc. as described above while the means for determining said biomarker on nucleic acid level includes appropriate compounds for amplification of said biomarker or for determining the presence of said biomarker using probes for example labeled probes accordingly.

In an embodiment, the use of the kit in a method according to the present invention relates to a kit comprising means for determining the level or amount of at least one of S100A12, S100A8, S100A9, KLRF1, PTGDR, MT-TC, RNU6-135P, JUP, AP001434.2, MYBL1, EEF1DP3, RP11-841O20.2, FGRBP2, SAMD3, MME-AS1, RP11-1143G9.4, MIR606, AFF3, GTSF1 L, ROR1, SLC9A7, RNU6-517P, MIR7641-2, KLHL14, CD200, FCRL5 and NAMPT in a sample, like a body fluid sample or body tissue sample, of the subject to be tested and instructions on how to use said test kit in a method according to any one of the preceding claims.

In an embodiment, said kit comprises means for determining the level or amount of at least one of S100A12, S100A8, S100A9, KLRF1, PTGDR, MT-TC, RNU6-135P, JUP, AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT on protein level, in particular, whereby said means are antibodies.

In another embodiment, the present invention relates to the use of a kit useful in a method according to the present invention, whereby said means for determining the level or amount of at least one of S100A12, S100A8, S100A9, KLRF1, PTGDR, MT-TC, RNU6-135P, JUP, AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT on protein level are antibodies, like monoclonal antibodies, and said method is an ELISA or lateral flow rapid test.

Moreover, the present invention relates to the use of a kit according to the present invention in a method as described herein for determining the amount or level of at least one of S100A12, S100A8, S100A9, KLRF1, PTGDR, MT-TC, RNU6-135P, JUP, AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT on the nucleic acid level, in particular, said kit is for use in amplification based methods including PCR.

It is clear that all methods described herein may be conducted in combination or in connection with other methods known in the art for bladder cancer detection. That is, it may be advantageous to complement the methods of the invention with established methods for bladder cancer identification or diagnosis.

The kits according to the present invention may comprise also other components like components for processing a blood sample. Such components for processing a blood sample may comprise a stabilizing level in certain embodiments as well as components for purification of nucleic acid molecules or peptides/proteins accordingly.

The kit may further incorporate means, namely components, like reactants for extraction isolation, concentration or purification of the nucleic acid molecules or protein/peptides to be determined. In further embodiments, the kit may also incorporate a sealable vessel for collection of a blood sample.

Moreover, suitable controls may be utilized in order to act as quality control for the methods to be included in the kit of the invention.

The kits of the invention may additionally include several buffers and/or other reactants for carrying out the claimed method of the invention as means. In a certain embodiment, the kit of the invention further comprises, consists essentially of or consist of nucleic acid amplification buffers including the necessary components like polymerases etc.

The above components present in the kit are all subsumed under the term "means". The skilled person is well aware of suitable "means" accordingly.

Of note, the term "means" and "components" are used herein interchangeably unless otherwise indicated.

Further, the methods described herein allows to determine the kind of treatment required for said subject. For example in case of high risk recurrence, prophylactic treatment may be effected. Further, depending on the diagnosis, the type of treatment including surgery and chemotherapy is selected. Further, the skilled artisan may identify that a change of the therapy regimen is required.

The present invention will be described further by way of examples without being limited thereto.

### Specimen and Data Collection

The selection of patients was based on the following inclusion criteria: the patient was diagnosed with Bladder Cancer (cystoscopic and histological evidence of Bladder Cancer). Recurrence-negative patients are defined as showing no cystoscopic or histological evidence of bladder cancer during monitoring. Recurrence-positive patients are defined as showing cystoscopic and histological evidence of bladder cancer during monitoring after treatment of de novo Bladder Cancer. New patients are bladder cancer patients with no previous history of bladder cancer. Healthy volunteers with no previous history of bladder cancer or any other cancer (Table 1). Average Age of positive cancer patients is 75.4 years. Average Age of recurrence-negative patients is 70.5 years. Average Age of new Bladder Cancer patients is 76.7 years. Average Age of Healthy volunteers is 68 years.

**Table 1. Summary of participant and specimen characteristics.**

| | | **No of patient TCC** | | | | | | **Muscle invasive** |
|---|---|---|---|---|---|---|---|---|
| **Patients** | **No of Patients** | **Total TCC** | **G 1 pTa** | **G2 pTa** | **G2 pT1** | **G3 pTa** | **G3 T1** | **G3 T2a** |
| **Recurrence-positive** | 17 | 15 | 4 | 9 | 1 | 0 | 1 | 2 |
| **Recurrence-negative** | 18 | 0 | 0 | 0 | 0 | 0 | 0 | 0 |
| **New Bladder Cancer patients** | 11 | 9 | 1 | 4 | 3 | 0 | 1 | 2 |
| **Health volunteers** | 29 | 0 | 0 | 0 | 0 | 0 | 0 | 0 |

All individuals gave informed consent for sample donation and the collection was approved by local ethical committee. Plasma from blood samples was collected from the same cohort patients except 4 patients and one healthy volunteer were missed. Plasma was obtained from cohort participant blood using BD Vacutainer^{®} Plasma Tubes (Heparin). Tubes containing 8-10 ml of blood were mixed for 30min at RT and centrifuged at 1600g for 10min at RT. Plasma was added to a fresh tube and centrifuged again at 1400g for 10min at RT. Plasma was then transferred to cryovials and stored at -80°C. Whole blood was collected using BD PAXgene™ Blood RNA Tubes. After the patient was bled the PAXgene™ Blood RNA Tubes were incubated at RT for 2hrs and then chilled on ice for 10mins. The tubes were then incubated at - 20°C overnight and then transferred to a storage box at -80°C.

### RNA and Array processing

Isolation of total RNA from clinical whole blood samples was performed according to the manufacturer's protocol using the PAXgene^{®} Blood RNA-Kit (PreAnalytiX). The total RNA was quantified by a Qubit™ Assay (Invitrogen) based on the manufacturer's standard protocols. The RNA integrity was determined by agarose gel electrophoresis. The RNA samples were processed using the GeneChip^{®} WT Plus Reagent Kit (Affymetrix) according to the manufacturer's protocol. In brief, the total RNA was first transcribed to double stranded cDNA, which was transcribed to cRNA. The cRNA was then synthesized into single stranded cDNA, which was fragmented and biotinylated. Finally the biotinylated single stranded cDNA was hybridized onto the whole transcript Affymetrix^{®} Human Gene 2.1 ST arrays, which cover a total of 40,716 annotated transcripts. After labeling with a streptavidin phycoerythrin conjugate the strips were washed and scanned by the Washing Station and the Imaging Station from the GeneAtlas^{®}System (Affymetrix). The probe cell intensity data (CEL) files were generated using the Command Console™ software (Affymetrix).

### Gene and Exon expression analysis

The CEL files were imported into Partek Genomics Suite version 6.6 (Partek Inc., St. Louis, MO, USA) for the gene and exon expression analysis using the Robust Multi-array Average (RMA) settings for normalization. A batch removal was performed to minimize the influence of external factors on the data. The lists of differentially expressed genes were created using analysis of variance (ANOVA) with an fdr (false discovery rate) corrected p-value <0.05 and a fold change > 1.5. The quality of the experiments was assessed on the basis of the QC metrics table and QC graphical report. Spearman's correlation was used as a similarity matrix to conduct average linkage hierarchical clustering. Differentially expressed genes were grouped into functional categories ranked according to their p-values using the Partek Gene Ontology (GO) Enrichment tool. Additionally the differentially expressed genes were plotted in a volcano plot. In the second analysis the CEL file were imported into DNASTAR^{®} ArrayStar^{®} (version 12.0) software (DNASTAR. Madison, WI) using again the RMA normalization. The lists of differentially expressed genes were generated using a Student's t-Test with a confidence interval >95% and a fold chance >1.5. Unannotated and duplicate probesets were removed from the lists.

### Quantitative Real-Time PCR (qPCR)

Expression of selected genes was verified performing a quantitative PCR (qPCR) using the housekeeping gene *GAPDH* as reference. The primers used are described in Table 2.

**Table 2. Primers used in the qPCR analysis.**

| **Gene Symbol** | **Type** | **Sequence (5'-3')** | **Amplicon size [bp]** |
|---|---|---|---|
| S100A12 | forward | GCA AAC ACC ATC AAG AAT ATC AA SEQ ID No.: 1 | 119 |
| | reverse | GCA ATG GCT ACC AGG GAT A SEQ ID No.:2 | |
| NAMPT | forward | TAT TGC CTT CGG TTC TGG TG SEQ ID No.:3 | 139 |
| | reverse | TTT GTT GGG ATC AGC AAC TG SEQ ID No.:4 | |
| S100A8 | forward | GCC AAG CCT AAC CGC TAT AA SEQ ID No.:5 | 82 |
| | reverse | CCC ACC AGG TCT TCT GAA AG SEQ ID No.:6 | |
| JUP | forward | AAC CAG GAG AGC AAG CTG AT SEQ ID No.:7 | 128 |
| | reverse | CAC ACG GAT AGC ACC TTG AG SEQ ID No.:8 | |
| KLRF1 | forward | ATG ACC AAC TTG AAA TGG CT SEQ ID No.:9 | 148 |
| | reverse | GGT CCC TTT ATG AAG AAT ATC TTT G SEQ ID No.:10 | |
| PTGDR | forward | CTC TGC CCG TAA TTT ATC GC SEQ ID No.:11 | 110 |
| | reverse | CAC AGA TAG AAA TCG CAA GGC SEQ ID No.:12 | |
| GAPDH | forward | GGA AGG TGA AGG TCG GAG TCA SEQ ID No.:13 | 101 |
| | reverse | GTC ATT GAT GGC AAC AAT ATC CAC T SEQ ID No.:14 | |

The analysis was performed in 2 µl containing each 10 µl 2x FastStart Essential DNA Master Mix (Roche), 2.4 µl H₂O(PCR grade), 8 µM of the respective forward and reverse primers and 5 mg/µl template DNA. As template DNA the single strand cDNA generated for the microarrays was used. No template was included in the negative control. PCR protocol comprised the following steps: denaturation at 95°C followed by 9 touch-down cycles with the annealing temperature decreasing 1°C per cycle from 61°C to 53°C. Then 31 cycles followed with 20 s at 95°C, 20 s at 53°C and 20 s at 72°C. Finally the DNA was denatured at 95°C for 20 s then at 55°C for 60 s and at 97°C for 1 s to generate the melting curves. The qPCR was performed on the Light Cycler® 96 (Roche) and the relative gene expression was determined using the Light Cycler® 96 software (version 1.1). Normalization was performed using the healthy samples as run calibrator. The normalized ratios were calculated as the target/reference ratio of the samples divided by the target/reference ratio at run calibrator.

### S100A12 quantitation in plasma samples using biolayer interferometry (BLI)

BLI experiments were conducted using an Octet QKe Instrument and high precision streptavidin biosensor (SAX), manufactured by ForteBio (Menlo Park, CA, USA). This system measures light interference on the surface of a fiber optic sensor, which is directly proportional to the thickness of molecules bound to the surface. Antibodies against the targets of interest are chemically tethered to the surface of the sensor using biotin-streptavidin interactions. Binding of S100A12 in the diluted plasma to the tethered antibodies results in thickening of the surface, this is monitored in real time. Purified rabbit anti-S100A12 polyclonal IgG were obtained from St. John's Laboratory (London, UK). Anti-S100A12 antibodies were biotinylated using Biotin Protein Labelling Kit (Roche) per the manufacturer's instructions. Sephadex G-25 columns (GE Healthcare) were then used to remove unreacted biotinylation reagent and buffer exchange into PBS. The biotinylated anti-S100A12 was immobilized on SAX biosensors at a single concentration of 10µg/ml for 40 min (online). To reduce the impact of non-specific binding to the sensor surface; the sensors were further blocked by incubating them in a solution of 0.1 % BSA in PBS for a minimum of 5 min. The regeneration of the sensors was performed with 10 mM glycine buffer (pH=2.2). All experiments were performed at 30 °C with an agitation set at 1000 rpm using solid black 96-well plates (Grieger Bio-One) with 10 min assay time (read time window) of dipping the prepared sensors in each well. The final volume for all the solutions was 200µl/well. Different concentrations of recombinant S100A12 (from 1857ng/ml to 0µg/ml) spiked in 1:12 diluted healthy human plasma to stablish the standard curve. The cohort plasma samples were all 1:12 diluted using PBS, pH 7.4 before applying in the assay plate. Data were analysed with the Octet System Data Analysis software v7.1 (ForteBio, Menlo Park, CA, USA), and the S100A12 concentration in plasma samples was obtained using calibration curve which set up using the BLI response (binding rate) to the spiked rS100A12 concentration. The GraphPad prism package was used in statistical calculations. To test the significance of differences between mean S100A12 concentrations in different patient groups we used an unpaired t-test with Welch's correction. Receiver operator characteristics (ROC) curves were generated for the S100A12 and the area under the curve tested for significance using an unpaired t-test.

### Results

### Microarray expression analysis of UCB patients

We carried out blood samples gene expression profiling in UCB cancer patients groups and/or in comparison to the healthy samples (Table 1). We first examined the numbers of genes detected under comparison of UCB patients and healthy volunteers. This comparison revealed a set of 14 differentially regulated genes. Of these genes 5 were upregulated in bladder cancer patients while 9 were downregulated (see Fig 1). The significance of regulated genes was set based on an fdr-corrected p-value <0.05 and a fold change >1.5. The most significantly upregulated genes included *RNASE2, RNU6-237P* and *S100A12,* while the most significantly downregulated genes comprised *IGLV3-21, IGHV1-2* and *TRAV13-1.*

Next, the UCB cohort patient's samples were divided into different groups according to the prognosis of the patients. New UCB cases were labeled "new positive", in case of a recurrence of the disease within 5 years after treatment the samples were assigned to the group "recurrent" and if a patient had a bladder history but was negative at the time the sample was taken, they were called "previously positive". The analysis of variance (ANOVA) is carried out between these groups resulted in a total of 127 differentially regulated genes. The comparison between the new positive samples and the negative control samples accounted for the majority of these genes showing 9 upregulated genes among the new positive patients and 38 downregulated genes. In total the comparison between the recurrent and the new positive samples revealed 38 differentially regulated genes.

Eighteen of these genes were exclusively found in this comparison as possible prognostic markers to distinguish recurrent cases from new UCB cases (Table 3).

**Table 3. Exclusive differentially regulated gene set between recurrent and new positive samples.**

| **Gene Symbol** | **Gene description** | **Fold change** | **p-value** |
|---|---|---|---|
| *AP001434.2* | lincRNA | 2.221 | <0.001 |
| *MYBL1* | Myb-related protein A | 1.67 | 0.00136 |
| *EEF1DP3* | pseudogene | 1.647 | <0.001 |
| *RP11-841020.2* | antisense RNA | 1.597 | <0.001 |
| *FGRBP2* | fibroblast growth factor binding protein 2 | 1.594 | <0.001 |
| *SAMD3* | Sterile alpha motif domain-containing protein 3 | 1.537 | <0.001 |
| *MME-AS1* | antisense RNA | 1.528 | <0.001 |
| *RP11-1143G9.4* | antisense RNA | 1.516 | <0.001 |
| *MIR606* | miRNA | 1.512 | <0.001 |
| *AFF3* | AF4/FMR2 family member 3 | -1.502 | 0.00106 |
| *GTSF1L* | Gametocyte-specific factor 1-like | -1.503 | <0.001 |
| *ROR1* | Inactive tyrosine-protein kinase transmembrane receptor ROR1 | -1.511 | <0.001 |
| *SLC9A7* | Sodium/hydrogen exchanger 7 | -1.541 | 0.00102 |
| *RNU6-517P* | snRNA | -1.563 | 0.00134 |
| *MIR7641-2* | miRNA | -1.591 | <0.001 |
| *KLHL14* | Kelch-like protein 14 | -1.635 | <0.001 |
| *CD200* | OX-2 membrane glycoprotein | -1.678 | <0.001 |
| *FCRL5* | Fc receptor-like protein 5 | -1.682 | <0.001 |

Data analysis was performed using the software Partek^{®} Genomic Suite (ANOVA; fdr-corrected p-value <0.05, fold change >1.5).

To identify an expression pattern that is related to UCB recurrence, we noticed that *PTGDR, KLRF1* and *MT-TC* genes were found to be significantly downregulated in new positive samples compared to negative and recurrent samples, while *RNU6-135P* was upregulated in new positive samples compared to previously positive and recurrent samples (Table 4).

**Table 4. Selected potential prognostics biomarkers**

| | Fold Change | | |
|---|---|---|---|
| Gene | New Positive vs. Healthy | Recurrent vs. New positive | New Positive vs. Previously positive |
| *KLRF1* (Killer cell lectin-like receptor subfamily F member 1) | -1.87 | 1.97 | -1.57 |
| *PTGDR* (Prostaglandin D2 receptor) | -1.71 | 1.73 | - |
| *MT-TC* (Mitochondrially encoded tRNA cysteine) | -1.91 | 2.04 | -1.8 |
| *RNU6-135P* (RNA, U6 small nuclear 135, pseudogene) | - | -2.65 | 1.98 |

Furthermore, to investigate the effects of risks groups in UCB, a model was built by using healthy, previously positive and dividing patients samples into risk groups according to stage and grade of the disease as defined previously (Kulkarni JN, Bakshi GK (2008), Indian J Urol 24: 68-71) (Table 5).

**Table 5. Division of samples into risk groups according to grade and stage of the tumor after Kulkarni and Bakshi**

| **Group** | **Grade and Stage** |
|---|---|
| High risk | G3 pTa; G3 pT1; G3 pT2 |
| Intermediate risk | G2 pTa; G2 pT1 |
| Low risk | G1 pTa; G1 pT1 |

ANOVA analysis carried out between these groups revealed no differentially regulated genes in the comparison between the low risk and negative samples. In the contrast between high risk and negative controls only the gene *JUP* was found to be significantly upregulated (fdr-corrected p-value <0.05; fold change >1.5) (Table 6).

**Table 6. Differentially regulated gene set in risk group analysis.**

| **Gene Symbol** | **Gene description** | **Fold change** | **p-value** | **Comparison** |
|---|---|---|---|---|
| *JUP* | Junction plakoglobin | 1,64 | <0,001 | High vs. Healthy |
| *RNU6-707P* | RNA, U6 small nuclear 707, pseudogene | 1,66 | 0,0012 | Intermediate vs. Healthy |
| *S100A12* | Calcium binding Protein S100A12 | 1,61 | 0,0016 | Intermediate vs. Healthy |
| *CLEC12A* | C-type lectin domain family 12 member A | 1,59 | 0,0125 | Intermediate vs. Healthy |
| *RNU6-237P* | RNA, U6 small nuclear 237, pseudogene | 1,58 | 0,0023 | Intermediate vs. Healthy |
| *IGHV1-2* | immunoglobulin heavy variable 1-2 | -1,51 | 0,0043 | Intermediate vs. Healthy |
| *TRAJ42* | T cell receptor alpha joining 42 | -1,52 | 0,0225 | Intermediate vs. Healthy |
| *TRAJ56* | T cell receptor alpha joining 5 | -1,53 | 0,0276 | Intermediate vs. Healthy |
| *IGKV1-17* | immunoglobulin kappa variable 1-17 | -1,55 | 0,0127 | Intermediate vs. Healthy |
| *TRAJ19* | T cell receptor alpha joining 19 | -1,56 | 0,0432 | Intermediate vs. Healthy |
| *SNORA5A* | small nucleolar RNA, H/ACA box 5A | -1,58 | 0,0032 | Intermediate vs. Healthy |
| *HIST1H3H* | histone H3.1 | -1,58 | 0,02897 | Intermediate vs. Healthy |
| *IGKV2-24* | immunoglobulin kappa variable 2-24 | -1,59 | 0,0209 | Intermediate vs. Healthy |
| *TRDJ4* | T cell receptor delta joining 4 | -1,63 | 0,0348 | Intermediate vs. Healthy |
| *IGHG1* | Ig gamma-1 chain C region | -1,63 | 0,0543 | Intermediate vs. Healthy |
| *IGLV1-40* | immunoglobulin lambda variable 1-40 | -1,64 | 0,0114 | Intermediate vs. Healthy |
| *TRDV1* | T cell receptor delta variable 1 | -1,66 | 0,0113 | Intermediate vs. Healthy |
| *RNU4ATAC* | RNA, U4atac small nuclear (U12-dependent splicing | -1,67 | 0,0575 | Intermediate vs. Healthy |
| *KLRC3* | NKG2-E type II integral membrane protein | -1,71 | 0,0046 | Intermediate vs. Healthy |
| *TRAJ17* | T cell receptor alpha joining 1 | -1,71 | 0,0015 | Intermediate vs. Healthy |
| *TRAJ29* | T cell receptor alpha joining 29 | -1,80 | 0,0013 | Intermediate vs. Healthy |

Further analysis between intermediate risk group and negative controls resulted in a total of 20 differentially regulated genes, 4 of which were upregulated while 16 are downregulated in patients. The most significantly upregulated genes were *RNU6-707P* and *S100A12. TRAJ29* and *TRAJ17* were the most significantly downregulated genes (Table 6).

### S100A12 is UCB relevant and its expression elevated in high-risk patients

Apparently, S100A12 is the major gene which differentially regulated in several analyses. This gene is significantly upregulated in the comparisons between patients and healthy volunteers as well as between intermediate risk patients and healthy volunteers. In both cases a fold change of 1.6 was associated to this particular gene. To achieve the goal of detecting potential biomarkers, these results were checked by analyzing the microarray data with the DNAStar ArrayStar software. A student's t-test was applied with a confidence interval of 95 % and a fold change >2.0. This analysis also showed a significant upregulation of *S100A12* in bladder cancer patients compared to healthy volunteers. In addition the analysis of the different risk groups displayed an expression pattern among these groups. The most significant upregulation was found among the high risk patients with a fold change of 3.15. Among the intermediate risk patients the fold change went down to 2.63 and dropped further to only 1.85 among the low risk patients. Interestingly in previously positive group S100A12 also was found to be upregulated (2.15 fold).

Moreover, in the ArrayStar analysis another two genes belong to the S100 family has been revealed to be differentially regulated in UCB patients compared to healthy volunteers. *S100A8* and *S100A9* were significantly upregulated with a fold change of 1.92 and 1.56 respectively. Another interesting gene showing a significant upregulation in the ArrayStar analysis, which had not been found in Partek was *NAMPT* with a fold change of 2.05.

To validate the results of the microarray analysis, real-time qPCR was performed for S100A12 and S100A8 using the housekeeping gene *GAPDH* as reference. The cDNA samples were pooled according to their risk and prognostic groups. The analysis of the risk pools resulted in an expression pattern for *S100A12* similar to the ArrayStar analysis. This gene was 2.1 fold upregulated in high risk patients and 1.8 fold upregulated in intermediate risk patients. In the low risk pool the normalized ratio dropped to 1.1 (Fig 2A). *S100A8* was shown to be 2 fold upregulated in intermediate risk patients, in high risk the fold change was 1.4 and low risk group showed a fold change of 1.2. The qRT-PCR analysis of the prognostic pools confirms also the upregulation patterns of *S100A12.* In new positive pool the fold change was found to be 2.1 compared to healthy individuals. The upregulation in the recurrent pool dropped to 1.7 and further to 1.3 in the previously positive pool. A similar regulation patterns was found for *S100A8* which was 1.9 fold upregulated in new positive patients but only 1.4 fold upregulated in recurrent patients. In previously positive patients the fold change dropped to 1.1 (Fig 2B).

### Plasma S100A12 concentrations are predictive for UCB

Given the mRNA expression of S100A12 in UCB patients and tissue sections, secretion of S100A12 by tissue cells in body fluids will signifying the uses of this marker as diagnosis indicator. Full length S100A12 protein could be detected in the plasma of cancer patients by western blotting (data not shown); to quantify the S100A12 concentration in UCB samples, the S100A12 was analysed in plasma samples using biolayer interferometry (BLI). We assessed plasma S100A12 levels in the same microarray cohort of patients and controls. We found S100A12 levels were significantly higher in those diagnosed with cancer compared with healthy volunteers, *p-value <* 0.0001. The mean of plasma S100A12 concentration in patients with UCB was 579.4 ± 30.58 ng/ml, whilst that for control subjects was 311.9 ± 37.03 ng/ml (Fig 3). Higher grade tumors (Grades 2 and 3) were associated with higher mean urinary S100A12 concentrations (618.3 ± 60.59 ng/ml for Grade 2 and 562.1 ± 88.46 ng/ml for Grade 3) than grade 1 tumors (434.5 ± 38.92 ng/ml) (Fig 4).

The receiver operating characteristic area under the curve (ROC AUC) for test performance was 0,869, with a sensitivity of 90.5% and a specificity of 75% (see Fig 3). The ROC analysis indicated that the optimal plasma S100A12 concentration threshold for cancer versus controls was 350ng/ml in order to maximize the sensitivity and specificity of the test. The area under the curve rises to 0.888 when comparing intermediate risk group of cancer patients to healthy group (Fig 4). While for high risk group the area under curve dropped down.

To test if S100A12 can also serve as recurrence and prognostic marker, the ROC analysis in study prognostics groups. The ROC analysis indicated that the S100A12 concentration could differentiate UCB-recurrent group and the previously positive group (recurrent-negative at sample) from new positive UCB patients with area under the curve rises of 0,793 and 0,725, respectively (Fig 5). These values are not affecting the ability of S100A12 to still be indicative for UCB new patients with area under the curve rises of 0.833 (Fig 5).

### Discussion

Bladder cancer is one of the most common neoplasm in industrialised countries, in terms of incidence. The worldwide UCB statics and the survival rate for muscle invasive bladder cancer patients which is only around 50%, highlight the fact that early detection and diagnosis of bladder cancer is critical for improving prognosis and overall survival rate. The recent treatment stream only curing a fraction of the patients and clearly has the best effect on the early diagnosed patients. Despite the large and growing list of candidate protein markers for UCB, but to date, no recommendation of routine uses of these markers. Clinicians need improved tools for selecting treatments and follow-up regimens for individual patients. Biomarkers identifying patients most likely to respond to chemotherapy for example would have enormous utility, not only reducing morbidity and improving quality of life but also significant cost savings to health providers. This is further exemplified, again in UCB, by the fact that chemotherapy may also be useful in a proportion of patients receiving chemotherapy in the neoadjuvant (i.e. before cystectomy) and adjuvant (post-surgery) settings as they are deemed to be patients at high risk of relapse, but no biomarkers can reliably identify individuals who would be most likely to benefit. Therefore, it is of paramount importance to identify novel and validated UCB biomarkers for the detection of disease and the UCB recurrence.

Different studies have focused on the global expression profiling of UCB using microarrays. The prediction of classes of bladder tumors using a limited set of genes could help in development of a clinical decision model. The main objective of the current study is to predict bladder cancer disease course identify markers that will predict the likelihood of progression in patients with high grade tumors. By utilizing gene expression profiling, we identified different genes as a signature biomarkers for UCB and tumor progression in risk and prognostics groups using a multiple group comparison. Initial comparison was performed on UCB patients against the healthy samples which served as control set. This comparison resulted in a set of 14 differentially regulated genes. One of these genes is S100A12 which upregulated in UCB patients (Fig 1). Subsequent grouping of the UCB patients according to their prognosis resulted in a set of 127 differentially regulated genes. Among this multigene expression signature in UCB blood samples, we can identify new potential biomarkers for the prognosis of bladder cancer. *KLRF1* encodes the killer cell lectin-like receptor F1, which is an activating C-type lectin-like receptor. It is expressed on human natural killer (NK) cells and subsets of T cells. It has been shown that a ligand of this receptor (activation-induced C-type lectin, AICL) is produced by hematopoetic as well as non hematopoetic tumor cells. Blocking of the interaction of AICL and *KLRF1* led to a partial inhibition of NK cell degranulation, showing that these receptors play an important role in the killing of tumor cell by NK-cells. A differential regulation of this gene in connection with cancer has not yet been described. Our microarray results however show that the expression of this gene in new positive patients is considerably stronger downregulated than in recurrent or previously positive bladder cancer patients.

*PTGDR* is also considerably higher downregulated in new positive patients than in recurrent and previously positive patients. This gene codes for the prostaglandin D₂ receptor, which is expressed in various cell types including cells of the immune system (NK-cells, dendritic cells) as well as cells of central nervous system and smooth muscle cells. The receptor is activated by prostaglandin D₂ which is involved in a variety of different processes like sleep, regulation of body temperature and release of hormones. It also inhibits the aggregation of platelets and promotes relaxation of smooth muscles. It has been shown that the expression of this gene is downregulated in colorectal adenocarcinomas and that there is a correlation between this dysregulation and disease progression as well as a hypermethylation of the gene. Such hypermethylations have also been demonstrated in the case of bladder cancer, the expression of the gene however has not been examined. As shown in Table 4 our analysis also showed other novel markers in UCB like *MT-TC* and *RNU6-135P* (RNA U6 small nuclear 135). *MT-TC* stands for the mitochondrial tRNA cysteine, which has not been connected to cancer so far. It has however been shown, that the expression of tRNA genes in general as well mitochondrial tRNA gene specifically are substantially upregulated in the case of breast cancer. These genes could therefore be potential cancer biomarkers.

For searching expression patterns between UCB samples based on risk classification the UCB samples were sorted according to clinical risk score. Our microarray analysis has shown *JUP* to be considerably upregulated in samples of high risk patients with high grade and stage bladder tumors. This gene encodes plakoglobin (also known as γ-catenin), which has been reported to be involved in the reduction of proliferation, migration and invasion of cells in vitro as well as the induction of apoptosis. *Rieger-Christ et al.* have reported plakoglobin as tumor suppressor in bladder cancer carcinoma and that silencing of this gene in late stage bladder cancer is associated with tumor progression. It has however also been demonstrated that wild-type and several amino-terminal mutated forms of plakoglobin had transforming activity on RK3E epithelial cells.

Characteristics of effective biomarkers include cancer-specific expression and release from tumors. One gene group that has recently been shown to have these properties is the S100 protein family and specially S100A12, which is expressed in different type of cancers. Recently, S100A12 has attracted substantial interest due to the roles in inflammation. In our analysis, S100A12 was found to be regulated in several contrasts among UCB samples and between risk groups. S100A12 has not been so far identified in other microarray expression studies as a gene specifically associated with UCB progression. S100A12 and S100A8 were selected for further investigation based on the results of UCB expression profiling. A clear correlation between the qPCR assays and the microarray data is observed, especially in case of S100A12. Anyhow, both genes were significant and independent prognostic indicators in patients with UCB. Our data suggest that these as molecular markers are more robust in identifying the patient subgroup with higher mortality risk than the grade 1. To investigate the eligibility of S100A12 as candidate body fluids biomarker, we decided to measure the concentration of S100A12 in UCB patient's plasma samples and compare it to healthy volunteer's samples. We found that S100A12 is associated with a sensitivity of 90.5% and specificity of 75% using the 350 ng/ml cut off. The maximum specificity that can be achieved using this assay is 100%, using a cut off at 880 ng/ml, but the resulting sensitivity is only 4.8%. The maximum sensitivity (100%) is achieved using a cut off at 230.4 ng/ml and the resulting specificity is 60%. The cut off value of 350 ng/ml was selected to give both high sensitivity and specificity. S100A12 might serve also as a prognostic marker because our study showed that overexpression of S100A12 protein was associated with recurrence of bladder cancer and with high-grade, high-stage and invasive tumors (Fig 4 and 5). Although S100A12 mRNA has been reported and linked to UCB, the present study is the first to indicate an increased expression of S100A12 protein and mRNA in human UCB and confirm the involvement of S100A12 in the progression of UCB. Hence, the utility of S100A12 at both mRNA and protein level as a remarkable biological marker of UCB detection and as prognostic marker is given. Furthermore, urine based biomarkers represents diagnostic tools and as predictors for later tumor recurrence.

The data revealed that the mRNA and protein expressions of S100A12 were significant. Thus, S100A12 is an independent prognostic marker for patients with UCB, which may predict the disease course of UCB patients and facilitate the clinical management of this cancer.

## Claims

1. A method for diagnosing or identifying bladder cancer in a subject comprising
a) determining the level or amount of at least one of S100A12, S100A8, S100A9, KLRF1, PTGDR, MT-TC, RNU6-135P, JUP, and NAMPT in a sample of said subject, and
b) diagnosing or identifying bladder cancer based on the level or amount of at least one of S100A12, S100A8, S100A9, KLRF1, PTGDR, MT-TC, RNU6-135P, JUP, and NAMPT.

2. A method for diagnosing or identifying bladder cancer in a subject not being diagnosed for bladder cancer before comprising
a) determining the level or amount of at least one of S100A12, S100A8, S100A9, KLRF1, PTGDR, MT-TC, and RNU6-135P in a sample of said patient; and
b) diagnosing or identifying for a first time bladder cancer in said subject based on the level or amount of at least one of S100A12, S100A8, S100A9, KLRF1, PTGDR, MT-TC, and RNU6-135P.

3. A method for identifying recurrence of bladder cancer in a subject previously diagnosed and optionally treated for bladder cancer comprising:
a) determining the level or amount of at least one of S100A12, KLRF1, PTGDR, MT-TC, and RNU6-135P AP001434.2, MYBL1, EEF1 DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 in a sample of said subject; and
b) identifying bladder cancer recurrence in said subject based on the level or amount of at least one of S100A12, KLRF1, PTGDR, MT-TC, and RNU6-135P.

4. A method for monitoring the progression of bladder cancer in a subject afflicted with bladder cancer, comprising:
a) detecting the level or amount of S100A12 and/or JUP in a first sample from a subject in a first point in time;
b) determining the level or amount of S100A12 and/or JUP in a second sample from the subject in a second point in time; and
c) comparing the level or amount of S100A12 and/or JUP determined in step a) to the level or amount detected in step b) or to a reference thereto for determining progression of bladder cancer based on the change of said level or amount.

5. A method for determining prognosis for survival of a subject afflicted with bladder cancer or for diagnosing or identifying high risk bladder cancer subjects, comprising the step of
a) determining the level or amount of at least one of S100A12 and JUP in a sample of said subject, and
b) classifying said subject into a group of subjects as having a decreased likelihood of survival or being a high risk bladder cancer subject based on the level or amount of at least one of S100A12 and JUP.

6. A method for monitoring the development, progress or recurrence of bladder cancer in a subject afflicted with or suffering previously from bladder cancer, comprising
a) determining the level or amount of S100A12 in a sample of said subject in a first time point and,
b) optionally, determining the level or amount of S100A12 in a second time point and
c) determining the development, progress or relapse of bladder cancer in said subject based on the level or amount determined in step a), optionally, compared to the level or amount detected in step b), or compared to a reference value.

7. A method for predicting a clinical outcome or for determining the treatment course in a subject afflicted with bladder cancer, comprising
a) determining the level or amount of at least one of S100A12, S100A8, S100A9, KLRF1, PTGDR, MT-TC, RNU6-135P, JUP, and NAMPT in at least one sample of the subject and
b) predicting the clinical outcome or determining the treatment course based on the level or amount of at least one of S100A12, S100A8, S100A9, KLRF1, PTGDR, MT-TC, RNU6-135P, JUP, and NAMPT present in said sample.

8. The method according to any one of the preceding claims wherein the sample from the subject is a sample of body fluid or body tissue of said subject, in particular, of urine or blood, like whole blood or serum or plasma.

9. The method according to any one of the preceding claims wherein the amount or level of S100A12 is determined.

10. The method according to any one of the preceding claims wherein the level or amount is determined by determining expression on protein level.

11. The method according to any one of the preceding claims wherein the level or amount of at least one of S100A12, S100A8, S100A9, KLRF1, PTGDR, MT-TC, RNU6-135P, JUP, AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT is determined by immunological methods, in particular, by ELISA or lateral flow rapid test.

12. The use of a kit in a method according to any one of the preceding claims, said kit comprises means for determining the level or amount of at least one of S100A12, S100A8, S100A9, KLRF1, PTGDR, MT-TC, RNU6-135P, JUP, AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT in a sample, like a body fluid sample or body tissue sample, of the subject to be tested and instructions on how to use said test kit in a method according to any one of the preceding claims.

13. The use of a kit according to claim 12 whereby said kit comprises means for determining the level or amount of at least one of S100A12, S100A8, S100A9, KLRF1, PTGDR, MT-TC, RNU6-135P, JUP, AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT on protein level, in particular, whereby said means are antibodies.

14. The use of a kit according to claim 12 or 13, whereby said means for determining the level or amount of at least one of S100A12, S100A8, S100A9, KLRF1, PTGDR, MT-TC, RNU6-135P, JUP, AP001434.2, MYBL1, EEF1DP3, RP11-841020.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT on protein level are antibodies, like monoclonal antibodies, and said method is an ELISA or lateral flow rapid test (point of care).

15. The use of a kit according to claim 12 for determining the amount or level of at least one of S100A12, S100A8, S100A9, KLRF1, PTGDR, MT-TC, RNU6-135P, JUP, AP001434.2, MYBL1, EEF1DP3, RP11-841O20.2, FGRBP2, SAMD3, MME-AS1, RP11-1143G9.4, MIR606, AFF3, GTSF1L, ROR1, SLC9A7, RNU6-517P, MIR7641-2, KLHL14, CD200, FCRL5 and NAMPT on the nucleic acid level, in particular, said kit is for use in amplification based methods including PCR.


SEQUENCE LISTING

<110>  Lionex GmbH

<120>  Novel human bladder cancer biomarkers and their diagnostic use

<130>  3441-027 EP-1

<160>  14

<170>  PatentIn version 3.5

<210>  1
<211>  23
<212>  DNA
<213>  artificial

<220>
<223>  oligonucleotide

<400>  1
gcaaacacca tcaagaatat caa                                              23


<210>  2
<211>  19
<212>  DNA
<213>  artificial

<220>
<223>  oligonucleotide

<400>  2
gcaatggcta ccagggata                                                   19


<210>  3
<211>  20
<212>  DNA
<213>  artificial

<220>
<223>  oligonucleotide

<400>  3
tattgccttc ggttctggtg                                                  20


<210>  4
<211>  20
<212>  DNA
<213>  artificial

<220>
<223>  oligonucleotide

<400>  4
tttgttggga tcagcaactg                                                  20


<210>  5
<211>  20
<212>  DNA

<213>  artificial

<220>
<223>  oligonucleotide

<400>  5
gccaagccta accgctataa                                                    20


<210>  6
<211>  20
<212>  DNA
<213>  artificial

<220>
<223>  oligonucleotide

<400>  6
cccaccaggt cttctgaaag                                                    20


<210>  7
<211>  20
<212>  DNA
<213>  artificial

<220>
<223>  oligonucleotide

<400>  7
aaccaggaga gcaagctgat                                                    20


<210>  8
<211>  20
<212>  DNA
<213>  artificial

<220>
<223>  oligonucleotide

<400>  8
cacacggata gcaccttgag                                                    20


<210>  9
<211>  20
<212>  DNA
<213>  artificial

<220>
<223>  oligonucleotide

<400>  9
atgaccaact tgaaatggct                                                    20


<210>  10
<211>  25
<212>  DNA
<213>  artificial

<220>

<223>  oligonucleotide

<400>  10
ggtcccttta tgaagaatat ctttg                                              25


<210>  11
<211>  20
<212>  DNA
<213>  artificial

<220>
<223>  oligonucleotide

<400>  11
ctctgcccgt aatttatcgc                                                    20


<210>  12
<211>  21
<212>  DNA
<213>  artificial

<220>
<223>  oligonucleotide

<400>  12
cacagataga aatcgcaagg c                                                  21


<210>  13
<211>  21
<212>  DNA
<213>  artificial

<220>
<223>  oligonucleotide

<400>  13
ggaaggtgaa ggtcggagtc a                                                  21


<210>  14
<211>  25
<212>  DNA
<213>  artificial

<220>
<223>  oligonucleotide

<400>  14
gtcattgatg gcaacaatat ccact                                              25